**Europäisches Patentamt**

**European Patent Office**

**Office européen des brevets**

(19)

(11) Numéro de publication : **0 148 715**
**B1**

(12)  # FASCICULE DE BREVET EUROPÉEN

(45) Date de publication du fascicule du brevet :
07.09.88

(51) Int. Cl.⁴ : **A 61 M   5/34**

(21) Numéro de dépôt : **84440011.9**

(22) Date de dépôt : **09.03.84**

(54) Seringue dentaire pour injections intra-ligamentaires.

(30) Priorité : **20.12.83 FR 8320528**

(43) Date de publication de la demande :
**17.07.85 Bulletin 85/29**

(45) Mention de la délivrance du brevet :
**07.09.88 Bulletin 88/36**

(84) Etats contractants désignés :
**AT BE CH DE GB IT LI LU NL SE**

(56) Documents cités :
**EP-A- 0 109 913**
**AT-B-   212 625**
**FR-A- 2 194 452**
**US-A- 1 569 961**
**US-A- 1 728 259**
**US-A- 2 020 111**
**US-A- 3 517 668**

(73) Titulaire : **MICRO-MEGA**
**5-12 rue du Tunnel**
**F-25000 Besancon (FR)**

(72) Inventeur : **Leonard, Henri**
**4 rue Jean de Vienne**
**F-25000 Besancon (FR)**

(74) Mandataire : **Poupon, Michel**
**3, rue Thiers**
**F-88000 Epinal (FR)**

## Description

L'invention a pour objet une seringue dentaire pour injections intra-ligamentaires, du type comportant un porte-aiguilles, un porte-carpules (5), une aiguille d'injection, et une carpule contenant le liquide à injecter (voir EP-A-109 913).

Dans ce cas, l'aiguille étant montée dans un support métallique dans l'axe de la tête d'injection, doit subir une flexion pour venir se placer dans l'axe de celle-ci. Pour assurer un guidage de la partie arrière de l'aiguille, en déterminant cette flexion, il est prévu dans la partie antérieure du support de la carpule une autre pièce métallique dont l'avant s'évase en forme de cône, de manière que la partie arrière de l'aiguille rencontrant ce cône, se trouve guidée comme par un entonnoir, vers l'axe de celui-ci, c'est-à-dire l'axe de la carpule.

Dans la pratique, il s'avère que si le support d'aiguille est très incliné, l'arrière de l'aiguille risque d'aborder l'entonnoir sous un angle trop important et au lieu d'être guidé comme prévu, se tord ou se coince contre le cône, ou tout au moins ne vient pas se placer axialement comme prévu.

La présente invention vise une variante éliminant cet inconvénient et ce risque.

A cet effet, dans la tête inclinée de la seringue est prévue une pièce unique comportant un canal de guidage et de maintien de l'aiguille, ce canal présentant la forme définitive que doit prendre l'aiguille après sa mise en place. Il en résulte que du début à la fin de la fixation du porte-aiguilles sur la tête de la seringue, l'aiguille est guidée et amenée à son emplacement et à sa forme voulue, sans risque d'erreurs.

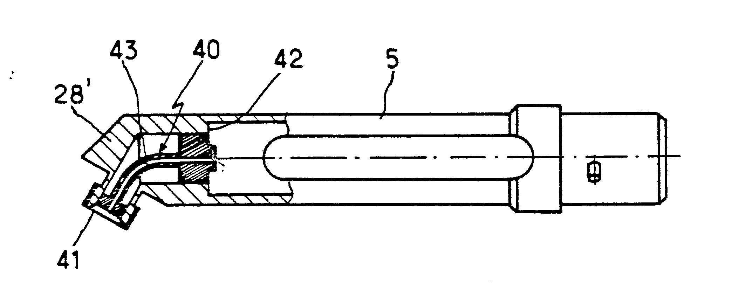

On va illustrer l'invention plus en détail en se référant au dessin annexé, dont la figure unique représente, en coupe suivant le plan vertical de symétrie de la seringue, le dispositif faisant l'objet de l'invention.

Sur ce dessin, le porte carpules ou douille 5 comporte un nez incliné 28. Au lieu d'une pièce s'évasant vers l'avant en forme d'entonnoir comme déjà connue, il est prévu selon l'invention une pièce 40, s'étendant depuis l'embouchure 41 du nez 28 jusqu'à la face avant 42 du porte-carpules. Cette pièce est arrondie pour suivre l'inclinaison du nez 28 par rapport à l'axe de la seringue, et elle est perforée axialement de bout en bout par un canal 43. Cette pièce 40 peut être réalisée en métal par tournage, perçage, puis courbure de manière à s'adapter exactement à l'angle entre les axes du nez 28 et de la douille 5, elle peut également être réalisée par moulage d'une matière plastique dure appropriée, de préférence auto-lubrifiante.

Un tel canal permet à l'aiguille, quand le porte-aiguilles est adapté sur la tête 41, de suivre d'elle-même le parcours qui amènera sa partie arrière dans l'axe de la carpule, sans risque de buter, de se tordre ou de se coincer comme c'était le cas dans les réalisations de l'art antérieur.

Au surplus, pour assurer l'étanchéité du système, on peut en envisager le collage, ce qui représente un avantage supplémentaire.

## Revendications

1. Seringue dentaire pour injections intra-ligamentaires, du type comportant un porte-aiguilles, un porte-carpules (5), une aiguille d'injection et une carpule contenant le liquide à injecter, caractérisée en ce que la portion avant (28) du porte-carpules est inclinée par rapport à l'axe de la seringue, et dans cette portion avant est montée une pièce (40) présentant un canal (43) axial arrondi, allant du porte-aiguilles à la carpule, en permettant d'introduire l'aiguille en assurant son guidage sur toute sa longueur.

2. Seringue selon la revendication 1, caractérisée en ce que ladite pièce est collée dans le nez, pour en assurer l'étanchéité.

3. Seringue selon les revendications 1 et 2, caractérisée en ce que ladite pièce est métallique et réalisée par tournage, perçage et courbure.

4. Seringue selon les revendications 1 et 3, caractérisée en ce que ladite pièce est réalisée par moulage d'une matière plastique, de préférence auto-lubrifiante.

## Claims

1. A dental syringe for intra-ligament injections of the type comprising a needle holder, a cartridge holder (5), an injection needle and a cartridge containing the liquid to be injected, characterised in that the front portion (28) of the cartridge holder is inclined to the axis of the syringe and, in this front portion, there is mounted a part (40) having a rounded axial channel (43) passing from the needle holder to the cartridge, while allowing the needle to be introduced and ensuring that it is guided over its entire length.

2. A syringe according to Claim 1, characterised in that said part is glued in the nose to ensure that it is sealed.

3. A syringe according to Claims 1 and 2, characterised in that said part is metallic and is produced by turning, perforation and bending.

4. A syringe according to Claims 1 and 3, characterised in that said part is produced by moulding a plastics material which is preferably self-lubricating. .

## Patentansprüche

1. Zahnärztliche Spritze für intra-ligamentäre Injektionen, mit einem Nadelhalter, einem Kartuschenhalter (5), einer Injektionsnadel und einer mit der zu injizierenden Flüssigkeit gefüllten Kartusche, dadurch gekennzeichnet, daß der Vorderteil (28) des Kartuschenhalters gegenüber der Spritzenachse geneigt ist, und daß in diesem ein Teil (40) eingefügt ist, das einen axial gerundeten Kanal (43) aufweist, der vom Nadelhalter zur Kartusche führt und der die Einführung der Nadel und ihre Führung entlang seiner ganzen Länge gewährleistet.

2. Spritze nach Anspruch 1, dadurch gekennzeichnet, daß das besagte Teil in die Nase eingeklebt ist, um deren Dichtheit zu gewährleisten.

3. Spritze nach den Ansprüchen 1 und 2, dadurch gekennzeichnet, daß das Teil aus Metall besteht und durch Drehen, Bohren und Biegen hergestellt ist.

4. Spritze nach den Ansprüchen 1 bis 3, dadurch gekennzeichnet, daß das Teil durch Spritzen aus einem möglichst selbstschmierenden Kunststoffmaterial besteht.